# EUROPEAN PATENT APPLICATION

(11) **EP 3 266 502 A1**
(43) Date of publication of application: **10.01.2018**
(21) Application number: 16177895.6
(22) Date of filing: 05.07.2016
(51) Int. Cl.: A61Q 5/00, A61Q 5/02, A61Q 5/10, A61Q 5/12, A61K 8/19, A46D 1/00

(54) **TREATMENT OF KERATINACEOUS FIBRES**

(71) Applicant: Unilever N.V., 3013 AL Rotterdam (NL)
(72) Inventor: MURALIDHARAN, Girish, Bangalore, 560 066 (IN); PRAMANIK, Amitava, Bangalore, 560 066 (IN); RANADE, Abhishek, Ahmedabad, Gujarat, 380 052 (IN); RAUT, Janhavi Sanjay, Bangalore, 560 066 (IN)
(74) Representative: Warner, Guy Jonathan

(57) **Abstract**

The present invention relates to a method of treating keratinaceous fibres, said method comprising contacting said keratinaceous fibres with a buffered aqueous silver solution containing Ag⁺ and having a buffered pH of 7 to 11.

The present method is capable of introducing exceptionally high levels of silver into keratinaceous fibres. In addition, the method offers the advantage that it does not require the use of strongly alkaline conditions and/or chemicals (e.g. peroxide or ammonia) that may cause damage to the keratinaceous fibres.

## Description

### TECHNICAL FIELD OF THE INVENTION

The present invention relates to a method of treating keratinaceous fibres, said method comprising contacting said keratinaceous fibres with an aqueous solution of silver salt. The present method may suitably be used to dye keratinaceous fibres and/or to impart anti-microbial properties thereto.

Human hair is an example of keratinaceous fibre that can suitably be treated by the present method.

### BACKGROUND OF THE INVENTION

In all mammals, hair develops as an epidermal structure from papillae deep in the skin and acquires characteristic patterns on the scalp, eyebrows, eyelashes, and elsewhere on the body.

Human hair is composed largely of keratin and consists of a narrow central medulla surrounded by a thick envelope (cortex) of elongate cells, which contain numerous melanin granules that determine the natural color. The hair is ensheathed in a multilayered cuticle of overlapping cells that become progressively imbricated (having edges overlapping in a regular arrangement) with continued growth. These cuticular cells are rich in cystine (disulfide bonds) and become rough or show a weathered appearance through exposure to environmental exposure or poor health.

Chemically, human hair contains approximately 85 percent protein, 7 percent water, 3 percent lipid , 4.7 percent protein-bound sulfur (as cystine), and low concentrations of trace minerals (e.g., iron, zinc, copper).

Melanin granules are secreted by melanocytes in the hair papilla and distributed to keratin in the hair cortex and inner layers of the hair sheath during normal development. Two main forms of melanin exist in human skin, i.e. eumelanin and phaeomelanin. Hair color is a balance between these two melanins.

A wide variety of dyes, dressings, and conditioners are available to men and women to enhance the color of hair or to alter its condition. Available hair dyes include:
- Minerals such as lead acetate, lead sulphide, silver nitrate, salts of bismuth, copper and cobalt (commonly called "gradual" colorants)
- Vegetable materials such as henna (flowers and leaves of Lawsonia inermis that contain acidic naphthoquinone, chamomile, and indigo)
- Synthetic dyes including a large number of organic dyes to provide permanent, semipermanent, temporary, or progressive color changes or to enhance natural colors

Metallic hair dyes tend to be long-lasting and are lost as hair grows and is shed naturally. Hair coloration is achieved as a gradual process through repeated application of rinses or pastes over several days.

Silver nitrate has been used to color hair brown-black without significant absorption of metal into the circulation; silver is deposited in the hair cortex as silver sulfide.

WO 2014/191115 describes a method of dyeing keratin fibers, said method comprising the application of an aqueous composition containing (a) at least one noble-metal compound selected from the salts and/or complex compounds of gold, silver, platinum, palladium, iridium, ruthenium, and/or rhodium and (b) at least one alkalizing agent. Examples 3 and 4 of this international patent application describe how white hair was dyed with an aqueous solution containing 0.1 wt.% AgNO₃ that having a pH of 12.5.

US 3,380,848 describes a method of producing solid polymeric material having bactericidal properties. Example 2 of this US patent describes the preparation of an aqueous silver nitrate solution by heating 1000 cc of demineralised water containing 1 g of silver nitrate to 95°C, followed b addition of 1% aqueous sodium hydroxide solution in an amount to results in a permanent slight cloudiness (about 3 cc.). Next, 50 grams of horsehair brush bristles were placed in the solution while maintaining the temperature and mixing for 30 minutes. The bristles became golden colored and were rendered bactericidal.

C.L. Gummer (Elucidating penetration pathways into the hair fiber using novel microscopic technique, J, Cosmet. Sci., (September/October 2001) 52, 265-280) describes immersion of hair fibers in aqueous silver nitrate for ten minutes at room temperature. Next, the solution was made alkaline with sodium hydroxide to precipitate silver hydroxide within the fiber, also at room temperature.

### SUMMARY OF THE INVENTION

The inventors have developed a method for treating keratinaceous fibres with aqueous silver solution that is very effective in that it can reproducibly achieve high levels of silver deposits within these fibres. The present method comprises the step of contacting keratinaceous fibres with a buffered aqueous solution of silver salt at a buffered pH of 7 to 11.

The inventors have discovered that the pH of the treatment solution has a great impact on, for instance, the amount of and rate at which silver that is deposited inside the keratinaceous fibres. In addition, the inventors have observed that the pH of the treatment solution is often affected by the keratinaceous fibres that it is applied to.

The present method offers the advantage that during the treatment of the keratinaceous fibres with the silver solution pH remains constant at an optimum level (pH 8-10). Thus, the optimum result can be achieved in a reliable manner.

Furthermore, the present method is capable of introducing exceptionally high levels of silver into keratinaceous fibres. Thus, the present method makes it possible to dye grey hair so that it attains an intense natural black color.

The present method does not require the use of strongly alkaline conditions and/or chemicals (e.g. peroxide or ammonia) that may cause damage to the keratinaceous fibres. Conventional hair dyeing methods involve bleaching of the hair using peroxide and alkaline solution. In addition to the desired decolourization of melanin, this treatment can result in hair damage. For example, weakening of the hair structure can occur due to oxidative breakage of the disulphide bonds in the hair fibres.

The present method can be used to dye keratineous fibres and/or to impart antimicrobial properties to these fibres. The method can also be used to prevent or reduce dandruff in hair.

The invention also relates to a kit that can suitably be used for dyeing keratinaceous fibres and a personal care product selected from a shampoo, a hair conditioner and a hair coloring product and an anti-dandruff product.

### DETAILED DESCRIPTION OF THE INVENTION

A first aspect of the present invention relates to a method of treating keratinaceous fibres, said method comprising contacting said keratinaceous fibres with a buffered aqueous silver solution containing Ag⁺ and having a buffered pH of 7 to 11.

The term "keratinaceous fibres" as used herein refers to fibres that mainly consist of keratin. Examples of keratinaceous fibres include hair (e.g. wool) and feathers.

The buffered pH of the silver solution that is employed in the present method refers to the buffered pH of the silver solution at the temperature at which it is brought into contact with the keratinaceous fibres.

The keratinaceous fibres that are treated in the present method are preferably selected from hair and feathers. More preferably, the keratinaceous fibres employed are hair, most preferably human hair.

In the present method the keratinaceous fibres are typically contacted with the buffered aqueous silver solution for at least 1 minute. More preferably, the fibres are contacted with the silver solution for 30 to 360 minutes,

The temperature at which the keratinaceous fibres are contacted with the buffered aqueous silver solution at a temperature preferably lies in the range of 10 to 80°C, more preferably in the range of 15 to 70°C and most preferably in the range of 25 to 60°C.

The buffered aqueous silver salt solution preferably has a molarity in the range of 0.1 to 100 mM Ag⁺, more preferably in the range of 0.5 to 20 mM Ag⁺

According to a particularly preferred embodiment, the buffered aqueous silver solution has a buffered pH in the range of 8-10.

The buffered aqueous silver solution that is used in the present method preferably is a solution of a water-soluble silver salt selected from silver nitrate, silver acetate and combinations thereof. Most preferably, the silver solution is a solution of silver nitrate.

The buffered aqueous silver solution may comprise different types of buffers. Examples of suitable buffers include carbonate, phosphate buffer and combinations thereof. Most preferably, the silver solution contains a carbonate-bicarbonate buffer. A carbonate bicarbonate buffer having a pH within the specified range can suitably be prepared by mixing an aqueous sodium carbonate solution (Na₂CO₃) with an aqueous sodium bicarbonate solution (NaHCO₃).

According to a particularly preferred embodiment, the combined concentration of carbonate and bicarbonate in the buffered aqueous silver solution is in the range of 0.02 - 10 mM, more preferably in the range of 0.04 - 4 mM.

The silver concentration in mM ([Ag⁺]), the carbonate concentration in mM ([CO₃²⁻]) and the bicarbonate concentration in mM ([HCO₃⁻]) meet the following condition: 0.1 ≤ [Ag⁺]/( ([CO₃²⁻]+[HCO₃⁻) ≤ 10 More preferably, these concentrations meet the following condition: 0.5 ≤ [Ag⁺] / ([CO₃²⁻]+[HCO₃⁻]) ≤ 2.

The buffered aqueous silver solution that is employed in the present method is preferably prepared by mixing an aqueous solution of silver salt with an aqueous buffer solution. Preferably, said aqueous silver salt solution has a molarity in the range of 0.1 to 100 mM Ag⁺, more preferably in the range of 0.5 to 20 mM Ag⁺. The aqueous buffer solution preferably has a pH in the range of 7 to 11, more preferably in the range of 8 to 10.

During the contacting of the keratinaceous fibres with the buffered aqueous silver solution typically silver containing nanoparticles having a diameter in the range of 1-200 nm, more preferably in the range of 5-50 nm are formed within the keratinaceous fibres.

The amount of silver that is introduced into the keratinaceous fibres during the contacting of the fibres with the buffered silver solution in the present method typically lies in the range of 0.1-250 mg silver per g of keratinaceous fibre. More preferably, the amount of silver absorbed lies in the range of 10-200 mg, most preferably of 20-150 mg per g of keratinaceous fibre.

The present method offers the advantage that it enables efficient incorporation of silver in keratinaceous fibres without the use of peroxide. This is an important benefit as peroxide is known to damage keratinaceous fibres, notably hair. Accordingly, in a preferred embodiment the buffered aqueous silver solution does not contain peroxide.

The method of the present invention also achieves good results without the use of ammonia. Consequently, in a further preferred embodiment, the buffered aqueous silver solution does not contain ammonia.

The inventors have unexpectedly discovered that keratinaceous fibres can advantageously be used to remove silver ions from aqueous liquid if a buffer has been added to said aqueous liquid to achieve a buffered pH in the range of 7 to 11. Thus, another aspect of the present invention relates to a method of removing silver from an aqueous liquid containing more than 1 mg/l dissolved Ag⁺, said method comprising:
- adding a buffer system to the aqueous liquid to achieve a buffered pH in the range of 7 to 11;
- adding keratinaceous fibres to the aqueous liquid before, after or simultaneous with the addition of the buffer system;
- allowing the keratinaceous fibres to bind silver from the aqueous liquid; and
- removing keratinaceous fibres containing bound silver from the aqueous liquid.

Typically, the aqueous liquid contains at least 3 mg/l dissolved Ag⁺, more preferably 5-100 mg/kg dissolved Ag⁺.

Preferably, at least 30 wt.%, more preferably at least 50 wt.% and most preferably at least 80 wt.% of the silver originally contained in the aqueous liquid is removed by the keratinaceous fibres in the present method.

The preferred conditions employed in this embodiment of the invention are the same as those described herein before in relation to the method of treating keratinaceous fibres.

Another aspect of the invention relates to the use of a buffered aqueous silver solution as defined herein for dyeing keratinaceous fibres.

Yet another aspect of the inventions relates to the use of a buffered aqueous silver solution as defined herein for preventing or reducing dandruff.

A further aspect of the invention relates to the use of a buffered aqueous silver solution as defined herein for imparting antimicrobial properties to keratinaceous fibres. Silver is a broad spectrum antimicrobial and antifungal.

The invention also provides a kit for treating keratinaceous fibres, said kit comprising:
- a separately packaged volume of an aqueous solution of silver salt having a molarity in the range of 0.1 to 100 mM, preferably in the range of 0.1 to 20 mM; and
- a separately packaged volume of an aqueous buffer solution having a pH in the range of 7 to11, preferably in the range of 8 to 10.

This kit can suitably be used, for instance, to dye keratinaceous fibres, notably human hair.

Typically, the kit comprises 1-1000 ml of the separately packaged volume of the solution of silver salt and 0.1-100 ml of the separate packaged volume of buffer solution.

Typically, the kit comprises instructions to mix a quantity of the aqueous solution of silver salt with a quantity of the aqueous alkaline buffer solutions and to contact the resulting aqueous mixture with keratinaceous fibres.

Finally, the invention provides a personal care product selected from a shampoo, a hair conditioner, a hair coloring product and an anti-dandruff product, said product having a water content of least 70 wt.% and containing 0.1 to 100 mmol dissolved Ag⁺ per kg of water and having a buffered pH of 7 to 11, preferably a buffered pH in the range of 8 to 10. This personal care product can suitably be used to impart anti-microbial properties to hair and/or to color hair and/or to reduce or prevent dandruff.

According to a particularly preferred embodiment, the personal care product contains 0.1 to 100 mmol dissolved Ag⁺ per kg of water, most preferably 0.5 to 20 mmol dissolved Ag⁺ per kg of water

The invention is further illustrated by the following non-limiting examples.

### EXAMPLES

### Example 1

European blonde hair fibres were washed with shampoo thoroughly to remove any impurities on the hair surface and left for drying.

A silver nitrate stock solution (1.5 M) concentration was prepared by dissolving 260 mg of silver nitrate in 1 ml de-ionized water.

A sodium hydroxide solution (-1.25 M) was prepared by dissolving 500 mg NaOH in 10 ml water.

A diluted silver nitrate solution was prepared by dissolving 1 ml in 500 ml water. The diluted silver nitrate solution was split into 5 fractions. Treatment solutions having a different pH (pH 2, 3, 5, 8, 10 and 12) were prepared by adjusting the pH of these fractions, using the sodium hydroxide solution and/or dilute nitric acid solution

The aforementioned hair fibres (300 mg of hair in each sample) were immersed in each of the treatment solutions. The solutions were kept for 26 days at 25°C in the dark to achieve equilibrium. Exposure to light was avoided as this may cause precipitation of silver.

After 26 days the silver content of the treated hair was determined using Inductively Coupled Plasma (ICP). The results are shown in Table 1.

**Table 1**

| **pH** | **mg Ag per g of hair** |
|---|---|
| 2 | 3 |
| 3 | 10 |
| 5 | 11 |
| 8 | 40 |
| 10 | 60 |
| 12 | 80 |

It was found that at pH greater than 11, non-uniform adsorption of silver onto hair fibres had occurred due to the formation of silver oxide/silver hydroxide precipitates in the solution. Also at pH 11, some non-uniform adsorption was observed, but much less pronounced than at pH 12.

### Example 2

Treatment solutions containing 3 mM AgNO₃ having a pH of 8.7 were prepared in the same way as described in Example 1, using either sodium hydroxide solution or carbonate-bicarbonate buffer to adjust the pH.

The carbonate-bicarbonate buffer was prepared by mixing 5 parts by weight of 5 mM aqueous sodium carbonate solution with 95 parts by weight of 40 mM aqueous sodium bicarbonate solution. The amount of buffering used was just sufficient to maintain the pH.

The hair fibres of Example 1 were immersed in each of the two treatment solutions and the solutions were kept for 35 days at 25°C in the dark.

After 35 days the silver content of the treated hairwas determined using ICP. The results are shown in Table 2.

**Table 2**

| **System** | **mg Ag per g of hair** | |
|---|---|---|
| | ***After 2 hours*** | ***Equilibrium (35 days)*** |
| NaOH | 8 | 70 |
| Buffer | 18 | 148 |

The color of the hair fibres was measured using a reflectometer. Thick hair strands (~ 300mg) were tied together and the values were measured. The results (L* and ΔE) are shown in Table 3. The L* value indicates whiteness index while the ΔE indicates the overall energy change in color space. High L* values indicate closeness to whiteness.

**Table 3**

| Samples | L* | ΔE |
|---|---|---|
| European blonde (Untreated) | 72.2 | |
| Treated with silver (NaOH) | 37.2 | 36.5 |
| Treated with silver (Buffer) | 21.9 | 54.9 |

This data indicates that treatment with silver solution in accordance with the present invention yields superior darkening of hair fiber. The level of coloring can be modulated by manipulating the silver content of the buffered silver solution.

### Example 3

25 mg of human hair that had been treated with the buffered silver solution of Example 2 and that contained approximately 100 mg silver per gram of hair was immersed in 50 ml demineralized water at 7 pH and 25°C. The silver content of the water was measured at regular intervals using ICP. The results are shown in Table 4.

**Table 4**

| **Time (min)** | **% silver leached** |
|---|---|
| 1 | 0.04 |
| 15 | 0.19 |
| 60 | 0.18 |
| 120 | 0.23 |
| 360 | 0.31 |
| 1,440 | 0.41 |
| 7,220 | 0.78 |
| 41,760 | 1.13 |

This data indicates that treatment with silver solution in accordance with the present invention yields silver-containing hair fibres that very slowly release silver over time. Such a slow release is desirable for a number of reasons. It means that the color of dyed fibres will hardly change over time and further that antimicrobial effects of the treatment can be sustained over a long period of time.

### Example 4

A 3 mM silver nitrate solution of pH 10 was prepared as described in Example 1 and was subsequently divided in equal amounts over two glass beakers.
Human hair fibres were immersed in the solution of one beaker while the other beaker was kept as it was. The beakers were kept at 25°C in the dark for 35 days. During that period the color of the hair fibres changed from blonde via dark brown to intense black. The silver content of the solutions was monitored over time. The initial silver weight is about 32 mg based on the silver nitrate used. The results are shown in Table 5.

**Table 5**

| **Time (min)** | **Amount of Silver in solution (mg)** | |
|---|---|---|
| | **With hair** | **Without hair** |
| 0.5 | 26.9 | 30.3 |
| 1 | 27.3 | 29.9 |
| 3 | 27.1 | 29.3 |
| 5 | 26.0 | 29.4 |
| 10 | 27.1 | 29.1 |
| 25 | 23.8 | 28.4 |
| 50 | 25.5 | - |
| 120 | 22.5 | - |
| 250 | 20.4 | - |
| 480 | 18.4 | - |
| 1,440 | 14.2 | - |
| 2,880 | 11.7 | - |
| 4,320 | 7.6 | 28.2 |
| 5,760 | 7.1 | - |
| 10,080 | 4.5 | - |
| 33,120 | 2.0 | - |

Also the silver content of the hair that was immersed in the silver solution was monitored over time. The results are shown in Table 6.

**Table 6**

| **Time (min)** | **Silver adsorbed onto hair (mg)** |
|---|---|
| 1 | 1.1 |
| 250 | 9.7 |
| 1,440 | 16.5 |
| 4,320 | 23.8 |
| 33,120 | 30.1 |

This data shows that silver ions are mopped by hair fibres and over time complete absorption onto hair can be achieved. This method also can help in scavenging silver ions.

## Claims

1. A method of treating keratinaceous fibres, said method comprising contacting said keratinaceous fibres with a buffered aqueous silver solution containing Ag⁺ and having a buffered pH of 7 to 11.

2. Method according to claim 1, wherein the buffered aqueous silver solution is a solution of a water-soluble silver salt selected from silver nitrate, silver acetate and combinations thereof.

3. Method according to any one of claims 1 or 2, wherein the buffered aqueous silver solution contains 0.1 to 100 mM Ag⁺, preferably 0.5 to 20 mM Ag⁺.

4. A method according to any of claims 1 to 3, wherein the buffered aqueous silver solution has a buffered pH of 8 to 10.

5. Method according to any of claims 1 to 4, wherein the keratinaceous fibres are contacted with the buffered aqueous silver solution for at least 1 minute, preferably between 30 to 360 minutes.

6. Method according to any of claims 1 to 5, wherein the keratinaceous fibres are contacted with the buffered aqueous silver solution at a temperature in the range of 10 to 80 °C , more preferably in the range of 15 to 70°C and most preferably in the range of 25 to 60°C.

7. Method according to any one of claims 1 to 6, wherein the buffered aqueous silver solution comprises a buffer selected from carbonate, phosphate and combinations thereof.

8. Method according to claim 7, wherein the buffered aqueous silver solution contains a carbonate-bicarbonate buffer.

9. Method according to claim 8, wherein combined concentration of carbonate and bicarbonate in the buffered aqueous silver nitrate solution is in the range of 0.02-10 mM, preferably 0.04-4 mM.

10. Method according to any of claims 1 to 9, wherein the treatment introduces 0.1-250 mg silver, preferably 10-200 mg silver per g of keratinaceous fibre and most preferably of 20 to150 mg per g of keratinaceous fibre.

11. Method according to any of claims 1 to 10, wherein the keratinaceous fibres are human hair.

12. A method of removing silver from an aqueous liquid containing at least 1 mg/l dissolved Ag⁺, said method comprising:
• adding a buffer system to the aqueous liquid to achieve a buffered pH in the range of 7 to 11;
• adding keratinaceous fibres to the aqueous liquid before, after or simultaneous with the addition of the buffer system;
• allowing the keratinaceous fibres to bind silver from the aqueous liquid; and
• removing keratinaceous fibres containing bound silver from the aqueous liquid.

13. Use of a buffered aqueous silver solution for dyeing keratinaceous fibres, for preventing or reducing dandruff, or for imparting antimicrobial properties to keratinaceous fibres, said solution containing 0.1 to 100 mM Ag⁺ and having a buffered pH of 7 to 11.

14. A kit for treating keratinaceous fibres, said kit comprising:
• a separately packaged volume of silver solution containing 0.1 to 100 mM of Ag⁺; and
• a separately packaged volume of an aqueous buffer solution having a pH in the range of 7 to11.

15. A personal care product selected from a shampoo, a hair conditioner, a hair coloring product and an anti-dandruff product, said product having a water content of least 70 wt.% and containing 0.1 to 100 mmol dissolved Ag⁺ per kg of water and having a buffered pH of 7 to 11.
